(19) Europäisches Patentamt
European Patent Office
Office européen des brevets

(11) EP 1 930 297 B1

(12) **EUROPEAN PATENT SPECIFICATION**

(45) Date of publication and mention of the grant of the patent:
**17.09.2014 Bulletin 2014/38**

(21) Application number: **06746456.0**

(22) Date of filing: **16.05.2006**

(51) Int Cl.:
***C01G 23/053*** (2006.01) ***A61K 8/00*** (2006.01)
***A61K 8/06*** (2006.01) ***A61K 8/19*** (2006.01)
***A61Q 17/04*** (2006.01) ***C08K 9/00*** (2006.01)
***C08L 101/00*** (2006.01) ***C09D 7/12*** (2006.01)

(86) International application number:
**PCT/JP2006/309742**

(87) International publication number:
**WO 2007/039953 (12.04.2007 Gazette 2007/15)**

(54) **PROCESS FOR PRODUCING FINE PARTICLE OF RUTILE-FORM TITANIUM OXIDE**

VERFAHREN ZUR HERSTELLUNG EINES FEINEN TEILCHENS AUS TITANOXID IN DER RUTILFORM

PROCEDE POUR LA PRODUCTION DE PARTICULE FINE D'OXYDE DE TITANE SOUS FORME DE RUTILE

(84) Designated Contracting States:
**DE ES FR GB IT NL**

(30) Priority: **30.09.2005 JP 2005289084**

(43) Date of publication of application:
**11.06.2008 Bulletin 2008/24**

(73) Proprietor: **SAKAI CHEMICAL INDUSTRY CO., LTD.**
**Osaka 590-8502 (JP)**

(72) Inventors:
- **MIZUTANI, Hideto**
**Fukushima 971-8183 (JP)**
- **TERABE, Atsuki**
**Fukushima 971-8183 (JP)**

(74) Representative: **Brunetti, Fabrizio et al**
**Cabinet Beau de Loménie**
**158, rue de l'Université**
**75340 Paris Cedex 07 (FR)**

(56) References cited:
**EP-A1- 1 167 462** **JP-A- 06 293 519**
**JP-A- 07 000 819** **JP-A- 10 152 323**
**JP-A- 2000 034 122** **JP-A- 2001 026 423**

- **HENGBO YIN ET AL: "Hydrothermal synthesis of nanosized anatase and rutile TiO2 using amorphous phase TiO2", JOURNAL OF MATERIALS CHEMISTRY, THE ROYAL SOCIETY OF CHEMISTRY, CAMBRIDGE, GB, vol. 11, no. 6, 9 April 2001 (2001-04-09), pages 1694-1703, XP002637773, ISSN: 0959-9428, DOI: 10.1039/B008974P [retrieved on 2001-04-09]**
- **JIANG B ET AL: "Size-controlled synthesis of anatase TiO2 nanoparticles by carboxylic acid group-containing organics", MATERIALS CHEMISTRY AND PHYSICS, ELSEVIER SA, SWITZERLAND, TAIWAN, REPUBLIC OF CHINA, vol. 92, no. 2-3, 15 August 2005 (2005-08-15), pages 595-599, XP004859847, ISSN: 0254-0584, DOI: 10.1016/J.MATCHEMPHYS.2005.02.007**
- **OJAMAE ET AL: "IR and quantum-chemical studies of carboxylic acid and glycine adsorption on rutile TiO2 nanoparticles", JOURNAL OF COLLOID AND INTERFACE SCIENCE, ACADEMIC PRESS, NEW YORK, NY, US, vol. 296, no. 1, 1 April 2006 (2006-04-01) , pages 71-78, XP005301033, ISSN: 0021-9797, DOI: 10.1016/J.JCIS.2005.08.037**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 930 297 B1

## Description

### TECHNICAL FIELD

The present invention relates to a method of producing a rutile titanium dioxide fine particle.

### BACKGROUND ART

Titanium dioxide fine particle is publicly known as an ultraviolet shielding material having transparency as described in the below Patent documents 1 and 2, and it is expected to be used to various applications. These applications can include, for example, an ultraviolet absorber for cosmetic compositions, additives for a resin composition and a coating composition, and inorganic coating compositions for forming an inorganic coat having photo catalytic performance.

However, a titanium dioxide fine particle having such a small particle size that an average primary particle diameter is 5 to 15 nm could not be obtained by the method described in the Patent document 1. The titanium dioxide fine particle described in the Patent document 2 is produced by reacting a reaction product of hydrous titanium dioxide and a basic sodium compound with hydrochloric acid in the presence of titanium trichloride. Since the titanium trichloride used here is expensive and this may be decomposed during storage because of low stability, it is not preferred. Therefore, a method of using an inexpensive and highly stable compound to produce a titanium dioxide fine particle has been required.

Patent document 1: JP-B-6-76215
Patent document 2: JP-A-2004-115342

### SUMMARY OF THE INVENTION

In view of the above state of the art, it is an object of the present invention to provide a method of producing a rutile titanium dioxide fine particle having a high ultraviolet protection property and high transparency for visible light.

The present invention pertains to a method of producing a rutile titanium dioxide fine particle, as defined in the claims.

In the method of producing a rutile titanium dioxide fine particle mentioned above,
the rutile titanium dioxide fine particle has an average primary particle diameter of 5 to 15 nm.

Although not being part of the claimed invention, also presently disclosed is a rutile titanium dioxide fine particle obtained by the method of producing a rutile titanium dioxide fine particle mentioned above.

The rutile titanium dioxide fine particle may have a high-density silica coating layer on the surface thereof.

The rutile titanium dioxide fine particle may have a coating layer of an organic silicon compound on the high-density silica coating layer.

Preferably, at least a part of the coating layer of an organic silicon compound is constituted of a branched organic silicon compound.

Although not being part of the claimed invention, also presently disclosed is a cosmetic composition containing the rutile titanium dioxide fine particle mentioned above.

Although not being part of the claimed invention, also presently disclosed is a coating composition containing the rutile titanium dioxide fine particle mentioned above.

Although not being part of the claimed invention, also presently disclosed is a resin composition containing the rutile titanium dioxide fine particle mentioned above.

Hereinafter, the present invention will be described in detail.

The present invention pertains to a method of producing a rutile titanium dioxide fine particle and a method of stably obtaining titanium dioxide in a fine particle form by using a carboxylic acid group-containing compound which is an inexpensive and highly stable compound. Therefore, the rutile titanium dioxide fine particle can be obtained at low cost. Particularly, the method of the present invention is an excellent method in that such a fine particle that an average primary particle diameter is 5 to 15 nm can be stably produced without using expensive and unstable titanium trichloride.

Incidentally, the above-mentioned average primary particle diameter can be determined by measuring a peak at a (110) plane of the rutile titanium dioxide fine particle (d=3.245) and applying the following Scherrer & Wilson's equation;

$$D = K \times \lambda/\beta\cos\theta\ .$$

wherein D represents a crystallite size, K represents a constant (assumed to be 1), $\beta$ represents an integral width, $\lambda$ represents a wavelength of an X-ray and $\theta$ represents a diffraction angle.

The rutile titanium dioxide fine particle obtained by the method of producing a rutile titanium dioxide fine particle of the present invention preferably has a specific surface area (by Brunauer-Emmerit-Teller method) of 100 to 200 $m^2$/g.

A first step in the method of producing a rutile titanium dioxide fine particle of the present invention is a step (step 1) of treating titanium dioxide hydrate with at least one of a basic compound selected from the group consisting of hydroxides of alkali metal and hydroxides of alkaline-earth metal.

Titanium dioxide hydrate can be obtained by the hydrolysis of a water-soluble titanium compound such as titanium sulfate, titanium chloride and the like. A method of hydrolysis is not particularly limited and a publicly known method can be applied. Among them, titanium dioxide hydrate obtained by thermal hydrolysis of titanium sulfate is preferred.

The above step (1) can be performed, for example, by adding the above basic compound to an aqueous suspension of the above titanium dioxide hydrate and treating (reacting) the resulting mixture for a predetermined time under the condition of a predetermined temperature.

A method of forming an aqueous suspension of the above titanium dioxide hydrate is not particularly limited and the aqueous suspension can be prepared by adding the above titanium dioxide hydrate to water and stirring the resulting mixture. The concentration of the suspension is not particularly limited but it is preferably, for example, such a concentration that the concentration of $TiO_2$ is 30 to 150 g/l in the suspension. Employing the above-mentioned concentration range allows a reaction (treatment) to proceed efficiently.

At least one of a basic compound selected from the group consisting of hydroxides of alkali metal and hydroxides of alkaline-earth metal used in the above step (1) is not particularly limited and it can include, for example, sodium hydroxide, potassiumhydroxide, magnesiumhydroxide and calcium hydroxide. The amount of the above basic compound to be added in the above step (1) is preferably such an amount that the concentration of the basic compound in a reactive (treating) suspension is 30 to 300 g/l.

The above step (1) is preferably performed at a reaction (treatment) temperature of 60 to 120°C. A reaction (treatment) time varies depending on the reaction (treatment) temperature, but a reaction time of 2 to 10 hours is preferred. A reaction (treatment) is preferablyperformedby adding an aqueous solution of sodium hydroxide, potassium hydroxide, magnesium hydroxide or calcium hydroxide to a suspension of titanium dioxide hydrate. The above-mentioned titanium dioxide hydrate treated with a basic compound can be obtained by cooling a reacted (treated) mixture after this reaction (treatment), neutralizing the reacted (treated) mixture with an inorganic acid such as hydrochloric acid as required, and then filtering and rinsing the reacted (treated) mixture.

A second step in the method of producing a rutile titanium dioxide fine particle of the present invention is a step (step 2) of treating the compound obtained by the step (1) with a carboxylic acid group-containing compound and an inorganic acid. In the production of the rutile titanium dioxide fine particle, a method of treating the compound obtained by the above step (1) with an inorganic acid is publicly known, but the method of the present invention is a method of using a carboxylic acid group-containing compound in addition to the inorganic acid. By using the carboxylic acid group-containing compound, a particle diameter can be controlled and a desired rutile titanium dioxide fine particle can be obtained.

The above-mentioned carboxylic acid group-containing compound is an organic compound having -COOH group. The above carboxylic acid group-containing compound is preferably a polycarboxylic acid having two or more carboxylic acid groups, more preferably at least two and at most 4 carboxylic acid groups. Since the above-mentioned polycarboxylic acid has the coordinating property to a metal atom, it is thought that the above polycarboxylic acid inhibits the coagulation between fine particles by this coordination and thereby the rutile titanium dioxide fine particle can be suitably obtained.

The above carboxylic acid group-containing compound is not particularly limited and examples of the compounds can include dicarboxylic acids such as oxalic acid, malonic acid, succinic acid, glutaric acid, adipic acid, propylmalonic acid and maleic acid; hydroxy multivalent carboxylic acids such as malic acid, tartaric acid and citric acid; aromatic polycarboxylic acids such as phthalic acid, isophthalic acid, hemimellitic acid and trimellitic acid; and ethylenediaminetetraacetic acid. Two or more species of these compounds may be simultaneously used in combination.

All or a part of the above carboxylic acid group-containing compound may be neutralized products of an organic compound having -COOH group (for example, an organic compound having -COONa group and the like). It is thought that -COOH groups derived from chemical equilibrium on mixing with an inorganic acid are produced even if the neutralized product is used on the treatment, therefore that the same treatment as one using the carboxylic acid group-containing compound according to the present invention may be performed.

The above-mentioned inorganic acid is not particularly limited and can include, for example, hydrochloric acid, sulfuric acid, and nitric acid. The above inorganic acid is preferably added in such a way that the concentration in a solution for the reaction (treatment) performing the step (2) is 0.5 to 2.5 mol/l, more preferably 0.8 to 1.4 mol/l.

The above-mentioned step (2) is preferably performed in such a manner that the compound obtained by the

above step (1) is suspended in pure water and treated under stirring with being heated as required. The inorganic acid is added after the addition of the carboxylic acid group-containing compound. The addition method includes a method (method 1) in which a carboxylic group-containing compound is added to a suspension of the compound obtained by the above step (1) and heating of this mixture is initiated, and an inorganic acid is added to this mixture when a mixture temperature reaches 60°C or higher, preferably 90°C or higher, and the resulting mixture is preferably stirred for 15 minutes to 5 hours, more preferably for 2 to 3 hours while maintaining a mixture temperatureAlthough not being part of the claimed invention, also presently disclosed is a method (method 2) in which a suspension of the compound obtained by the above step (1) is heated, and to this suspension, an inorganic acid is added when a temperature of the suspension reaches 60°C or higher, preferably 90°C or higher, and to this, a carboxylic acid group-containing compound is added after a lapse of 10 to 15 minutes from the addition of the inorganic acid, and the resultingmixture is preferably stirred for 15 minutes to 5 hours, more preferably for 2 to 3 hours while maintaining a mixture temperature. By performing theses methods, suitable rutile titanium dioxide in fine particle form can be obtained.

When the above step (2) is performed according to the above method 1, the above carboxylic acid group-containing compound is used in an amount 0.25 to 1.5 mole% with respect to 100 mole% of $TiO_2$, and more preferably used in an amount 0.4 to 0.8 mole%. When the amount of the carboxylic acid group-containing compound to be added is less than 0.25 mole%, the growth of a particle proceeds and a particle having a desired particle size may not be obtained, and when the amount of the carboxylic acid group-containing compound to be added is more than 1.5 mole%, the formation of a rutile-type particle does not proceed and an anatase-type particle may be produced.

When the above step (2) is performed according to the above method 2, the above carboxylic acid group-containing compound is preferably used in an amount 1.6 to 4.0 mole% with respect to 100 mole% of $TiO_2$, and more preferably used in an amount 2.0 to 2.4 mole%. When the amount of the carboxylic acid group-containing compound to be added is less than 1.6 mole%, the growth of a particle proceeds and a particle having a desired particle size may not be obtained, and when the amount of the carboxylic acid group-containing compound to be added is more than 4.0 mole%, the formation of a rutile-type particle does not proceed and an anatase-type particle may be produced, and an effect does not become better even though the amount of the carboxylic acid group-containing compound to be added is more than 4.0 mole% and this case is economically disadvantageous. Further, when the carboxylic acid group-containing compound is added before a lapse of 10 minutes from the addition of the inorganic acid, the formation of a rutile-type particle does not proceed and an anatase-type particle may be produced, and when the carboxylic acid group-containing compound is added after a lapse of more than 15 minutes from the addition of the inorganic acid, the growth of a particle proceeds excessively and a particle having a desired particle size may not be obtained.

In the above step (2), it is preferred that after the completion of a reaction (treatment), a reacted (treated) mixture is cooled and further neutralized in such a way that a pH falls within a range of 5.0 to 10.0. The above neutralization can be performed with an alkaline compound such as an aqueous solution of sodiumhydroxide or aqueous ammonia. Desired rutile titanium dioxide fine particle can be separated by filtering and rinsing after the neutralization.

Although not being part of the claimed invention, presently disclosed is also the rutile titanium dioxide fine particle thus obtained. The rutile titanium dioxide fine particle can be used for an ultraviolet absorber for cosmetic compositions, additives for a resin composition and a coating composition, and inorganic coating compositions for forming an inorganic coat having photo catalytic performance.

As is known, titaniumdioxide has photo catalytic activity. Since the photo catalytic activity take place at the surface of the titanium dioxide particle, the titanium dioxide particle becomes a particle having high photo catalytic activity when it has a small particle size as with the rutile titanium dioxide fine particle presently disclosed. When the titanium dioxide particle is used for applications utilizing the photo catalytic activity like inorganic coating compositions for forming an inorganic coat having photo catalytic performance, it is an advantageous property to have such high photo catalytic activity.

On the other hand, when the rutile titanium dioxide fine particle presently disclosed is used for an application of cosmetic compositions, or additives for a resin composition and a coating composition containing an organic resin, it is preferred to suppress surface activity by forming the high-density silica coating layer on its surface because the high photo catalytic activity is an undesirable property.

Since titanium dioxide generally has high surface activity, for example when it is mixed in a cosmetic composition, it causes problems that it makes an oil material, a perfume, a coloring material, an organic ultraviolet absorber or another additives, having been mixed in a cosmetic composition, chemically deteriorate, causes the discoloration and the off-flavor of mixed compounds, and varies the viscosity of the cosmetic composition in the case where the cosmetic composition is liquid. Further, when titanium dioxide with further smaller particle size is used, there is a problem that the above-mentioned problem becomes more noticeable since a specific surface area increases and the surface activity becomes high.

When the titanium dioxide fine particle is used for cosmetics, it is important that the surface of the titanium dioxide fine particle is not exposed to the outside so that organic compounds and the titanium dioxide, which are used in combination in the cosmetic composition, do not come into direct contact with each other in order to control the surface

activity of the titanium dioxide fine particle with reliability. Considering the above object, a material for coating has to be inactive even when it is in contact with an external substance, and a coating substance has to have a certain thickness and has to be tight. For example, if the density of the coating layer is low, external substances may penetrate into the surface of the particles through fine pores in the coating layer. Thus, the coating layer is required to be tight.

Further, when a titaniumdioxide fine particle is dispersed in an oil material and irradiated with sunlight, the titanium dioxide fine particle absorbed ultraviolet rays is discolored black-and-blue. However, the cosmetic composition presently disclosed , containing the above-mentioned specific rutile titanium dioxide fine particle, is not discolored black-and-blue. And, when a titanium dioxide fine particle is dispersed in white Vaseline and irradiated with sunlight, the vaseline is discolored yellow, but when the rutile titanium dioxide fine particle prepared by the above method is used, the vaseline is not discolored yellow. From these results, it is apparent that in an ultraviolet protective cosmetic composition mixed with the above rutile titanium dioxide fine particle, the surface activity is inhibited and the stability is excellent.

As a material constituting the coating layer, aluminum and zirconium compounds can be given other than silica from conventional technical findings, but it is difficult to form a tight coating layer of hydroxide or hydrous oxide of aluminum or zirconium in liquid. If powder of particles is calcined after forming the coating layer, the coating layer can be tighter, but if powder of particles is calcined, a state of fine particles cannot be maintained due to fusion between particles.

And, the high-density silica coating layer is preferably a layer in which amounts of components to be used other than silica are reduced. More specifically, it is preferred that the contents of elements other than silicon and oxygen in the high-density silica coating layer are less than 30 %. In a surface coating of pigment, a mixed coat such as silica-alumina or silica-zirconia is often formed. However, since the rutile titanium dioxide fine particle presently disclosed has an extremely small particle diameter and therefore has very high surface activity, it is preferred to form the high-density silica coating layer which is a tighter coating in order to completely suppress the surface activity.

The above-mentioned high-density silica coating layer is a silica layer formed more tightly than normal on the surface of the rutile titanium dioxide fine particle and it is preferably formed densely to the level by which the photo catalytic activity of titanium dioxide can be adequately inhibited. Such a high-density silica coating layer can includes, for example, acoatinglayer (a coating layer 1) formedbyaddingwater-soluble silicate to an aqueous suspension of the rutile titanium dioxide fine particle and adding acid to this mixture to neutralize the suspension, and a coating layer (a coating layer 2) formed by simultaneously adding water-soluble silicate and acid to the aqueous suspension of the rutile titanium dioxide fine particle while keeping a basic property and then further adding acid to neutralize the suspension.

In the formation of the above coating layer 1, first, the water-soluble silicate is added to the aqueous suspension of the rutile titanium dioxide fine particle. The concentration of the rutile titanium dioxide fine particle in the above-mentioned aqueous suspension preferably ranges from 50 to 250 g/l. The above-mentioned water-soluble silicate is not particularly limited and for example, sodium silicate, potassium silicate and the like can be used. An amount of the above water-soluble silicate to be added is preferably 10 to 100 weight % in terms of $SiO_2$ with respect to titanium dioxide. And, the rutile titanium dioxide fine particle is preferably prepared by milling crude titanium dioxide well with a mill such as a sand mill before treating.

When an amount of the above water-soluble silicate is less than 10 weight % in terms of $SiO_2$ there may be cases where the surface of the titanium dioxide fine particle cannot be completely coated, and when it is more than 100 weight %, the ability to shield ultraviolet rays may be deteriorated because of reduction in the titanium dioxide content.

The subsequent neutralization step is a step of forming a silica coating layer by adding acid gradually to the above aqueous suspension to neutralize. The neutralization step is preferably performed by adding acid with the expenditure of time of 30 minutes or more while maintaining a suspension temperature at 60°C or higher. The neutralization is preferably continued until a pH falls within a range of 6.0 to 8.0. The acid used for the above neutralization is not particularly limited and includes, for example, inorganic acids such as sulfuric acid and the like, and organic acids such as acetic acid, oxalic acid and the like can be used.

In the formation of the above coating layer 1, the condition of temperature and the condition of time (a period of time) for adding a neutralizing agent in the above neutralization step are important. That is, it is preferred that the neutralizing agent is added with the expenditure of time of 30 minutes or more, preferably 40 minutes or more, and more preferably 60 minutes or more while maintaining a suspension temperature in this step at 60°C or higher, preferably 80°C or higher and the suspension is neutralized until a pH falls within a range of 6.0 to 8.0.

In the formation of the above coating layer 2, first, the water-soluble silicate and the acid are added to the aqueous suspension of the rutile titanium dioxide fine particle. The concentration of the rutile titanium dioxide fine particle in the aqueous suspension preferably ranges from 50 to 250 g/l. The water-soluble silicate is not particularly limited and for example, sodium silicate, potassium silicate and the like can be used. An amount of the above water-soluble silicate to be added is preferably 1 to 50 weight % in terms of $SiO_2$ with respect to titanium dioxide. The above acid is not particularly limited and includes, for example, inorganic acids such as sulfuric acid and the like, and organic acids such as acetic acid, oxalic acid and the like can be used. The water-soluble silicate and the acid are preferably simultaneously added with the expenditure of time of 40 minutes or more while maintaining a suspension temperature and a pH within

ranges of 60°C or higher and 9 to 10.5, respectively. More preferably, the temperature during the addition is 80°C or higher and the time for addition is 60 minutes or more. Further, an amount of the above acid to be added is appropriately selected in such a way that a pH can be maintained within a range of 9 to 10.5.

The subsequent neutralization step is a step of forming a silica coating layer by adding acid to the above aqueous suspension to neutralize. The neutralization step is preferably continued until a pH falls within a range of 6.0 to 8.0. The acid used for the above neutralization is not particularly limited and for example, inorganic acids such as sulfuric acid and the like, and organic acids such as acetic acid, oxalic acid and the like can be used.

The rutile titanium dioxide fine particle having the high-density silica coating layer more preferably has the high-density silica coating layer in an amount of 1 to 50 weight % with respect to titanium dioxide on the surface thereof.

The rutile titanium dioxide fine particle having the above high-density silica coating layer may be a substance in which surface treatment by an organic silicon compound is further applied onto the high-density silica coating layer (a substance having a coating layer of an organic silicon compound). By applying the surface treatment by an organic silicon compound, dispersibility in an oil material in the cosmetic composition and water repellency can be enhanced. In the above surface treatment, it is preferred, generally, to allow an organic silicon compound in an amount of 1 to 20 weight % with respect to titanium dioxide to adhere to the high-density silica coating layer. An amount of 3 to 10 weight % is more preferred. When the amount of the above organic silicon compound is less than 1 weight % with respect to titanium dioxide, an effect of improving the dispersibility in the oil material in the cosmetic composition is poor, and on the other hand when the amount of the above organic silicon compound is more than 20 weight %, the dispersibility in the oil material in the cosmetic composition and the water repellency become saturated and this is economically disadvantageous. Examples of the above organic silicon compounds can include dimethylpolysiloxane, methylhydrogen polysiloxane, and branched silicone. These compounds may be used alone or in combination of two or more species. If they are used in combination, they may be used as a mixture of two kinds of the organosilicon compounds in a single step surface treatment, or may be used in a two-step treatment with each organosilicon compound in each step.

When the rutile titanium dioxide fine particle presently disclosed is used for an application of cosmetic compositions, it is preferred that at least a part of the above organic silicon compound is a branched organic silicon compound, and it is particularly preferred that at least a part of the above organic silicon compound is a branched silicone. The above branched silicone is silicone of a two-dimensional structure, which has a side chain consisting of a polysiloxane skeleton. The above branched silicone is a preferred one in that by use of this silicone, a feeling during use becomes better and a sense of use of a cosmetic composition is improved.

As the above branched silicone, for example, compounds like a substance disclosed in JP-A-2002-154917 can be employed. More specifically, there can be suitably used (i) a branched polysiloxane compound comprising a unit of $[R^1{}_3SiO_{1/2}]_k$ and a unit of $[R^1SiO_{3/2}]_1$, in which 1/k is 0.3 to 1.5, and (ii) a cyclic polysiloxane compound expressed by the following mean composition formula:

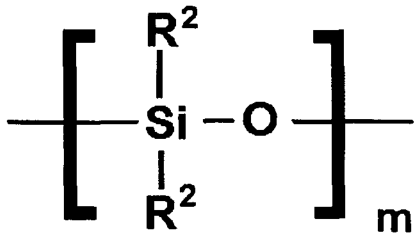

and/or (iii) a branched polysiloxane compound formed by polymerizing a polysiloxane compound expressed by the following mean composition formula:

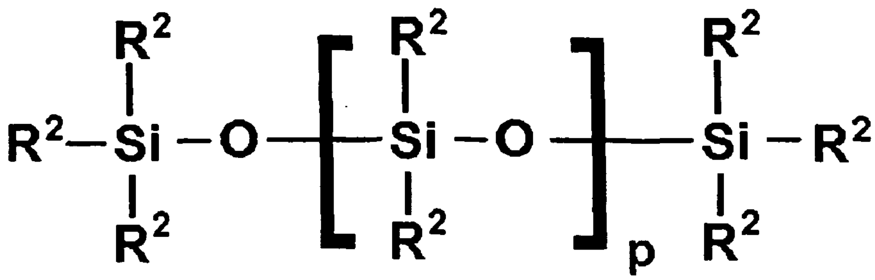

using an acid catalyst or an alkaline catalyst, wherein $R^1$ may be the same or different and is selected from a hydrogen atom, a hydroxyl group, an alkyl group having 1 to 30 carbon atoms, an aryl group, an aralkyl group, an alkyl group

having a substituent of fluorine, an alkyl group having a substituent of an amino group and an alkyl group having a substituent of a carboxyl group, and an alkoxy group having 1 to 6 carbon atoms, and $R^2$ may be the same or different and is selected from a hydrogen atom, an alkyl group having 1 to 30 carbon atoms, an aryl group, an aralkyl group, an alkyl group having a substituent of fluorine, an alkyl group having a substituent of an amino group and an alkyl group having a substituent of a carboxyl group, m is an integer of 3 to 10 and p is an integer of 0 to 100.

As abranchedorganic silicon compound, which is obtainable by the above-mentioned production method, for example, commercially available one can be employed, and as a commercially available one, for example, KF-9908, KF-9909 (trade name, both produced by Shin-Etsu Chemical Co., Ltd.) and the like can be suitably used. When the branched organic silicon compound is used, this compound may be used alone or maybe used in combination with a straight chain organic silicon compound such as dimethylpolysiloxane or methylhydrogen polysiloxane. This combined use is preferred in that balanced both properties of the sense of use as a cosmetic composition and the dispersibility in the oil material can be attained by the combined use. And, a method of surface treatment by the organic silicon compound may be a method of performing the surface treatment in one step using a mixture of two species of organic silicon compounds, or a method of performing the surface treatment in two steps using each of two species of organic silicon compounds. When the surface treatment is performed in two steps, it is preferred to perform the treatment by the branched organic silicon compound after the treatment by the straight chain organic silicon compound from the viewpoint that rutile titanium dioxide fine particle, which is excellent in both properties of the sense of use and the dispersibility, can be obtained.

The above-mentioned rutile titanium dioxide fine particle can be suitably used for cosmetic compositions, and a cosmetic composition containing the above-mentioned rutile titanium dioxide fine particle is also presently disclosed. The cosmetic composition preferably contains 1 to 80 weight % of the above-mentioned rutile titanium dioxide fine particle. When the content of the rutile titanium dioxide fine particle is less than 1 weight %, the effect of shielding ultraviolet rays cannot be adequately attained, and when the content is 80 weight % or more, it may become difficult to maintain the stability of a cosmetic composition. The above lower limit of the content is more preferably 5 weight % and the upper limit is more preferably 50 weight %.

The rutile titanium dioxide fine particle coated with the above organic silicon compound is preferred in that it is easy to make its formulation uniform on the side of an oil-base composition when it is processed into a emulsion of water in oil type. Further, when the ultraviolet protective cosmetic composition is applied to a skin, it is preferred that the cosmetic composition can be maintained on the skin for a long time by preventing the above rutile titanium dioxide fine particle from running down due to sweat from a human body. It is preferred to perform the surface treatment by the above organic silicon compound since high water repellency is attained.

And, the above cosmetic composition is often mixed with a silicone oil material, and an organic silicon compound like the one has a high affinity for the above silicone oil material and excellent dispersion stability. In addition to this, particularly, an organic silicon compound having a hydrogen group such as methylhydrogen polysiloxane, dimethylpolysiloxane-methylhydrogen polysiloxane copolymer or like, or a reactive organic silicon compounds having an alkoxyl group contributes to double inactivation of the surface activity together with a coating layer by hydroxide or hydrous oxide of silicon by eliminating an OH group which becomes a cause of the surface activity by reacting with the OH group of a powder surface to chemically bond. As a result, the surface activity of the rutile titanium dioxide fine particle can be controlled in a lower level, and high dispersibility is provided when the titanium oxide is mixed in cosmetic compositions. At the time of use, long life UV shielding effect can be maintained due to water repellency.

The cosmetic composition presently disclosed preferably further contains a volatile silicone oil. The titanium dioxide particle surface treated with the high-density silica coating layer and the organic silicon compound can be processed into a cosmetic composition having extremely high dispersibility by using it in combination with the volatile silicone oil. The volatile silicone oil is not particularly limited and publicly known substances such as decamethylcyclopentasiloxane, octamethylcyclotetrasiloxane and the like can be used. These substances may be used alone or in arbitrary combination of two or more species. Such a volatile silicone oil can be mixed in the cosmetic composition presently disclosed in an amount 5 to 70 weight % with respect to the total amount of the composition, preferably 10 to 40 weight %.

In the cosmetic composition presently disclosed, any aqueous component and oil-base component which can be used in cosmetics field may be used in combination in addition to the components constituting the above mixture. The aqueous component and oil-base component are not particularly limited, and for example, an oil substance, a surfactant, a moisturizing agent, a higher alcohol, a metal ion blocking agent, a natural or synthetic polymer, a water-soluble or oil-soluble polymer, an ultraviolet absorber, an extract, an inorganic or organic pigment, an inorganic or organic clay mineral, an inorganic and organic pigment treated with metal soap or silicone, a coloring agent such as an organic dye, an antiseptic agent, an antioxidant, a coloring matter, a thickner, a pH control agent, a perfume, a cooling agent, an antiperspirant, a fungicide and a skin activator may be contained. Specifically, it is possible that one or more species of components described below are mixed in an arbitrary proportions and a desired cosmetic composition is produced according to normal methods. Amounts of the components to be mixed are not particularly limited as long as they are

within a range in which an effect presently disclosed is not impaired.

The above-mentioned oil substance is not particularly limited and examples of the oil substances can include avocado oil, camellia oil, turtle oil, macadamia nut oil, corn oil, mink oil, olive oil, rape oil, egg oil, sesame oil, apricot kernel oil, wheat germ oil, camellia sasanqua oil, castor oil, linseed oil, safflower oil, cotton seed oil, perilla oil, soybean oil, peanut oil, tea oil, kaya oil, rice bran oil, china wood oil, Japanese wood oil, jojoba oil, germ oil, tri glycerin, glycerin triethylhexanoate, glycerin triisopalmitate, cocoa butter, palmoil, horse oil, hydrogenatedpalmoil, palmoil, beef tallow, mutton tallow, hydrogenated tallow, palm kernel oil, lard, beef bone fat, Japan wax-kernel oil, hydrogenated oil, beef leg oil, Japan wax, hydrogenated castor oil, beeswax, candelilla wax, cotton wax, carnauba wax, bayberry wax, purified insect wax, spermaceti, montanwax, bran wax, lanolin, kapok wax, acetylated lanolin, lanolin oil, cane wax, isopropyl lanolate, hexyl laurate, reduced lanolin, jojoba wax, hard lanolin, shellac wax, polyoxyethylene (POE) lanolin alcohol ether, POE lanolin alcohol acetate, POE cholesterol ether, polyethyleneglycol lanolate, POE hydrogenated lanolin alcohol ether, liquid paraffin, ozokerite, pristine, paraffin, ceresin, squalane, Vaseline and microcrystalline wax.

A lipophilic nonionic surfactant is not particularly limited and it includes, for example, sorbitan fatty acid esters such as sorbit an monooleate, sorbitan monoisostearate, sorbitan monolaurate, sorbitan monopalmitate, sorbitan monostearate, sorbitan sesquioleate, sorbitan triaoleate, penta-2-ethylhexanoyl diglycerol sorbitan and tetra-2-ethylhexanoyl diglycerol sorbitan, glycerin polyglycerin fatty acids such as cotton oil fatty acid monoglycerin, glycerin monoerucate, glyceryl sesquioleate, glyceryl monostearate, glycerin $\alpha,\alpha'$-oleate pyroglutamate and glyceryl monostearate monomalate, propylene glycol fatty acid esters such as propylene glycol monostearate, and hydrogenated castor oil derivative, and glycerin alkyl ether.

A hydrophilic nonionic surfactant is not particularly limited and it includes, for example, POE sorbitan fatty acid esters such as POE sorbitan monooleate, POE-sorbitan monostearate, POE-sorbitan monooleate and POE-sorbitan tetraoleate, POE sorbit fatty acid esters such as POE sorbit monolaurate, POE-sorbit monooleate, POE-sorbitpentaoleate and POE-sorbit monostearate, POE glycerin fatty acid esters such as POE-glycerin monostearate, POE-glycerin monoisostearate and POE-glycerin triisostearate, POE fatty acid esters such as POE monooleate, POE distearate, POE monodioleate and ethylene glycol distearate, POE alkyl ethers such as POE lauryl ether, POE oleyl ether, POE stearyl ether, POE behenyl ether, POE 2-octyldodecyl ether and POE cholestanol ether POE alkylphenyl ethers such as POE octylphenyl ether, POE nonylphenyl ether and POE dinonylphenyl ether, pluronic types such as pluronic, POE POP alkyl ethers such as POE POP cetyl ether, POE POP 2-decyltetradecyl ether, POE POP monobutyl ether, POE POP hydrogenated lanolin and POE POP glycerin ether, tetra-POE tetra-POP ethylenediamine condensates such as tetronic, derivatives of POE castor oil and POE hydrogenated castor oil such as POE castor oil, POE hydrogenated castor oil, POE hydrogenated castor oil monoisostearate, POE hydrogenated castor oil triisostearate, POE hydrogenated castor oil monopyroglutamate monoisostearate diester and POE hydrogenated castor oil maleate, POE bees wax lanolin derivative such as POE sorbit bees wax, alkanol amide such as palm oil fatty acid diethanol amide, lauric acid monoethanol amide and fatty acid isopropanol amide, POE propylene glycol fatty acid ester, POE alkyl amine, POE fatty acid amide, sucrose fatty acid ester, POE nonylphenyl formaldehyde condensate, alkylethoxydimethylamine oxide, and trioleyl phosphate.

As other surfactants, anionic surfactants such as fatty acid soap, higher alkyl sulfate ester, POE lauryl sulfate triethanolamine and alkyl ether sulfate ester, cationic surfactants such as alkyltrimethylammonium salt, alkylpyridinium salt, alkyl quaternary ammonium salt, alkyldimethybenzylmmonium salt, POE alkylamine, alkyl amine salt and polyamine fatty acid derivative, and amphoteric surfactants such as imidazoline-base amphoteric surfactant and betaine-base amphoteric surfactant may be mixed to such the extent that there is not a problem for the stability and the irritancy to skin.

The above-mentioned moisturizing agent is not particularly limited and examples of the moisturizing agents can include xylitol, sorbitol, maltitol, chondroitin sulfate, hyaluronic acid, mucoitinsulfuric acid, charoninic acid, atelocollagen, cholesteryl-12-hydroxystearate, sodiumlactate, bile acid salt, dl-pyrrolidonecarboxylic acid salt, short chain soluble collagen, PO (EO) adduct of diglycerin, extract of Rosa roxburghii, extract of achillea millefolium and extract of melilot.

The above-mentioned higher alcohol is not particularly limited and examples of the alcohols can include straight alcohols such as lauryl alcohol, cetyl alcohol, stearyl alcohol, behenyl alcohol, myristyl alcohol, oleyl alcohol and cetstearyl alcohol, andbranched chain alcohols such asmonostearyl glyceryl ether (batyl alcohol), 2-decyltetradecynol, lanolin alcohol, cholesterol, phytosterol, hexyldodecanol, isostearyl alcohol and octyldodecanol.

A metal ion blocking agent is not particularly limited and examples of the agents can include 1-hydroxyethane-1,1-diphosphonic acid, tetrasodium 1-hydroxyethane-1,1-diphosphonate, sodium citrate, sodium polyphosphate, sodiummetaphosphate, gluconic acid, phosphoric acid, citric acid, ascorbic acid, succinic acid and edetic acid.

The above-mentioned natural water-soluble polymer is not particularly limited and examples of the polymers can include vegetable polymers such as arabic gum, tragacanth gum, galactan, guar gum, carob gum, karaya gum, carrageenan gum, pectin, agar, quince seed (quince), algae colloid (brown alga extract), starch (rice, corn, potato, wheat) and glycyrrhizic acid, microbial polymers such as xanthan gum, dextran, succinoglycan and pullulan, and zoogenic polymers such as collagen, casein, albumin and gelatin.

A semi-synthetic water-soluble polymer is not particularly limited and examples of the polymers can include

starch polymers such as carboxymethylated starch and methylhydroxypropyl starch, cellulose polymer such as methyl cellulose, nitrocellulose, ethyl cellulose, methylhydroxypropyl cellulose, hydroxyethyl cellulose, cellulose sulfate sodium, hydroxypropyl cellulose, sodium carboxymethyl cellulose (CMC), crystalline cellulose and cellulose powder, and alginic acid polymers such as sodium alginate and propylene glycol alginate.

The above-mentioned synthetic water-soluble polymer is not particularly limited and examples of the polymers can include vinyl polymers such as polyvinyl alcohol, polyvinyl methyl ether and polyvinyl pyrrolidone, polyoxyethylene polymers such as polyethylene glycol 20000, polyethylene glycol 40000 and polyethylene glycol 60000, a copolymer of polyoxyethylene and polyoxypropylene, acrylic polymers such as sodium polyacrylate, polyethyl acrylate and polyacrylamide, polyethyleneimine, and cationic polymer.

An inorganic water-soluble polymer is not particularly limited and examples of the polymers can include bentonite, AlMg silicate (veegum), laponite, hectorite and silicic anhydride.

The above-mentioned ultraviolet absorber is not particularly limited and examples of the absorbers include benzoic acid ultraviolet absorbers such as p-aminobenzoic acid (hereinafter, abbreviated as PABA), PABA monoglycerin ester, N, N-dipropoxy PABA ethyl ester, N, N-diethoxy PABA ethyl ester, N, N-dimethyl PABA ethyl ester and N, N-dimethyl PABA butyl ester; anthranilic acid ultraviolet absorbers such as homomenthyl-N-acetyl anthranilate; salicylic acid ultraviolet absorbers such as amyl salicylate, menthyl salicylate, homomenthyl salicylate, octyl salicylate, phenyl salicylate, benzyl salicylate and p-isopropanolphenyl salicylate; cinnamic acid ultraviolet absorbers such as octyl cinnamate, ethyl-4-isopropyl cinnamate, methyl-2,5-diisopropylcinnamate, ethyl-2,4-diisopropyl cinnamate, methyl-2,4-diisopropyl cinnamate, propyl-p-methoxy cinnamate, isopropyl-p-methoxy cinnamate, isoamyl-p-methoxy cinnamate, 2-ethoxyethyl-p-methoxy cinnamate, cyclohexyl-p-methoxy cinnamate, ethyl-$\alpha$-cyano-$\beta$-phenyl cinnamate, 2-ethylhexyl-$\alpha$-cyano-$\beta$-phenyl cinnamate and glyceryl mono-2-ethylhexanoyl-dimethoxycinnamate; benzophenone ultraviolet absorbers such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone and 4-hydroxy-3-carboxybenzophenone; and 3-(4'-methylbenzylidene)-d,1-camphor, 3-benzylidene-d,1-camphor, urocanic acid, ethyl urocanate, 2-phenyl-5-methylbenzoxazole, 2,2'-hydroxy-5-methylphenylbenzotriazole, 2-(2'-hydroxy-5'-tert-octylphenyl)benzotriazole, 2-(2'-hydroxy-5'-methylphenyl)benzotriazole, dibenzalazin, dianisoyl methane, 4-methoxy-4'-tert-butyldibenzoylmethane, and 5-(3,3-dimethyl-2-norbornylidene)-3-pentan-2-one.

Other agents are not particularly limited and example of the agents can include vitamins such vitamin A oil, retinol, retinol palmitate, inositol, pyridoxine hydrochloride, benzyl nicotinate, amide nicotinate, DL-$\alpha$-tocopherol nicotinate, magnesium asrcorbate phosphate, 2-O-$\alpha$-D-glucopyranosyl-L-ascorbic acid, vitamin D2 (ergocalciferol), dl-$\alpha$-tocopherol, dl-$\alpha$-tocopherol acetate, pantothenic acid and biotin; hormones such as estradiol and ethinylestradiol; amino acids such as arginine, aspartic acid, cystine, cysteine, methionine, serine, leucine and tryptophan; anti-inflammatories such as allantoin and azulene, whitening agents such as arbutin; astringent such as zinc oxide and tannic acid; refrigerants such as L-menthol and camphor, sulfur, lysozyme hydrochloride, and pyridoxine chloride.

The above-mentioned various extracts are not particularly limited and examples of the extracts can include a variegated chameleon plant extract, a phellodendron bark extract, a melilot extract, a white deadnettle extract, a licorice extract, a peony extract, a soapwort extract, a dishcloth gourd extract, a quina extract, a strawberry geranium extract, a ku shen extract, a spatterdock extract, a fennel extract, a primrose extract, a rose extract, a rehmannia glutinosa extract, a lemon extract, a shikon extract, a aloe extract, a sweet flag extract, a eucalyptus extract, a field horsetail extract, a sage extract, a garden thyme extract, a tea extract, a sea weed extract, a cucumber extract, a clove extract, a framboise extract, a melissa extract, a carrot extract, a horse chestnut extract, a peach extract, a peach leaf extract, a mulberry extract, a cropweed extract, a hamamelin extract, a placenta extract, a sweetbread extract, a silk extract and a licorice extract.

The cosmetic composition presently disclosed may be used as a cosmetic composition like sun block cosmetic compositions such as a sunscreen, make up bases and foundations. And, it can be used in any form of an oil-base cosmetic composition, an aqueous cosmetic composition, a cosmetic composition of oil in water type, and a cosmetic composition of water in oil type, but it is preferably used as an emulsified cosmetic composition of water in oil type because this type is excellent in water resistance. The cosmetic composition presently disclosed can be processed into any one of the forms such as powder, pressed powder, cake form, emulsion, solution and gel.

In accordance with the present invention, rutile titanium dioxide fine particle having a high ultraviolet protection property and high transparency for visible light can be produced atlowprice. And, by further treating the above titanium dioxide particle, it is possible to suppress photo catalytic activity and provide water resistance and a good feeling without frictional feeling. The cosmetic composition containing the above-mentioned treated titanium dioxide particle performs efficacy of having the stability of a composition by suppressing photo catalytic activity and an excellent feeling.

BRIEF DESCRIPTION OF THE DRAWINGS

Fig. 1 is a view showing the discoloration of Vaseline in irradiating ultraviolet rays to a composition consisting of the titanium dioxide fine particle obtained in Examples 5 and 6 and Comparative Example 4 and the Vaseline.

BEST MODE FOR CARRYING OUT THE INVENTION

Hereinafter, the present invention will be described in more detail by way of examples, but the present invention is not limited to these examples.

<Treatment of titanium dioxide hydrate by a basic compound (step (1))>

To 10 liters of an aqueous suspension (concentration of $TiO_2$ 100 g/l) formed by suspending titanium dioxide hydrate in water, 30 liters of an aqueous solution (concentration 10 mole/l) of sodium hydroxide was added while stirring, and the resulting mixture was heated to 90°C and aged for 5 hours, and then neutralized with hydrochloric acid, filtered and rinsed. In addition, as the titanium dioxide hydrate in the above reaction (treatment), titanium dioxide hydrate obtained by thermally hydrolyzing an aqueous solution of titanium sulfate according to a publicly known method was used.

Example 1

A titanium compound treated with a base was suspended in pure water in such a way that the concentration of $TiO_2$ was 20 g/l, and to this suspension, citric acid was added in an amount 0.4 mole% with respect to 100 mole% of $TiO_2$ under stirring and a temperature of the resulting mixture was raised. When a temperature of the mixture reached 95°C, concentrated hydrochloric acid was added in such a way that the concentration of hydrochloric acid was 30 g/l and the mixture was stirred for 3 hours while maintaining the mixture temperature.

After cooling the mixture, the mixture was neutralized so as to be a pH 7 . 5 with an aqueous solution of sodium hydroxide, filtered and rinsed to obtain a cake of titanium dioxide fine particle.

Example 2 (not part of the invention)

A cake of titanium dioxide fine particle was obtained by following the same procedure as in Example 1 except for adding citric acid in an amount 2 mole% with respect to 100 mole% of $TiO_2$ after a lapse of 10 minutes from the addition of hydrochloric acid without adding citric acid before the addition of hydrochloric acid.

Example 3

A cake of titanium dioxide fine particle was obtained by following the same procedure as in Example 1 except for adding malic acid in an amount 0.4 mole% with respect to 100 mole% of $TiO_2$ in place of citric acid.

Example 4

A cake of titanium dioxide fine particle was obtained by following the same procedure as in Example 1 except for adding succinic acid in an amount 0.4 mole% with respect to 100 mole% of $TiO_2$ in place of citric acid.

Comparative Example 1

A cake of titanium dioxide fine particle was obtained by following the same procedure as in Example 1 except for not adding citric acid.

Comparative Example 2

A cake of titanium dioxide fine particle was obtained by following the same procedure as in Example 1 except for adding citric acid in an amount 2.0 mole% with respect to 100 mole% of $TiO_2$.

Comparative Example 3

A cake of titanium dioxide fine particle was obtained by following the same procedure as in Example 1 except for adding citric acid in an amount 0.2 mole% with respect to 100 mole% of $TiO_2$.

The cakes of titanium dioxide fine particle obtained in Examples 1 to 4 and Comparative Examples 1 to 3 described above were dried at 105°C for 3 hours, and measurement of a specific surface area by Brunauer-Emmerit-Teller (BET) method and X-ray diffraction was carried out. It was identified whether each particle is a rutile-type or an anatase-type based on the results of the measurement, and an average primary particle diameter was measured on a rutile-type particle. The results of measurement are shown in Table 1. In addition, measurement of a specific surface area by BET method was carried out using 4-SORB U2 (model name, manufactured by YUASA-IONICS COMPANY, LIMITED) and measurement of X-ray diffraction was carried out using JDX-3530 Type (model name, manufactured by JEOL DATUM LTD.).

Table 1

| | Specific surface area by BET method ($m^2/g$) | Average primary particle diameter (nm) | Crystal type |
|---|---|---|---|
| Example 1 | 142 | 10 | rutile |
| Example 2 | 146 | 9 | rutile |
| Example 3 | 130 | 11 | rutile |
| Example 4 | 111 | 13 | rutile |
| Comparative Example 1 | 84 | 18 | rutile |
| Comparative Example 2 | 286 | - | anatase |
| Comparative Example 3 | 65 | 20 | rutile |

From the results in Table 1, it is apparent that in accordance with the method of the present invention, the titanium dioxide fine particle which has a fine particle diameter and is a rutile-type can be suitably obtained.

<Evaluation of titanium dioxide surface treated>

Example 5 (not part of the invention)

The cake of titanium dioxide fine particle of Example 1 was processed into an aqueous suspension in which the concentration of $TiO_2$ was 75 g/l. This suspension was heated to 80°C and to this, an aqueous solution of sodium silicate in an amount of 30 weight % in terms of $SiO_2$ with respect to 100 weight % of the above titanium dioxide fine particle was added under stirring. After aging the mixture for 10 minutes, sulfuric acid was added over 180 minutes while stirring to neutralize the mixture to yield a pH of 7.0. After aging the mixture for 30 minutes, the resulting suspension was filtered and rinsed, and then dried by heat at 130°C for 5 hours. The dried article thus obtainedwas milled with a jet mill to obtain titaniumdioxide fine particle having a high-density silica coating layer on the surface thereof (an amount of the high-density silica coating layer was 29 weight % with respect to 100 weight % of the titanium dioxide).

In addition, an amount of a coating was measured with an X-ray fluorescence analyzer (3270 type manufactured by Rigaku Corporation)(same applies to the following).

Example 6 (not part of the invention)

The cake of the titanium dioxide fine particle of Example 1 was processed into an aqueous suspension in which the concentration of $TiO_2$ was 75 g/l. This suspension was heated to 80°C and to this, an aqueous solution of sodium silicate in an amount of 60 weight % in terms of $SiO_2$ with respect to 100 weight % of the above titanium dioxide fine particle was added under stirring. After aging the mixture for 10 minutes, sulfuric acid was added over 360 minutes while stirring to neutralize the mixture to yield a pH of 7.0. After aging the mixture for 30 minutes, the resulting suspension was filtered and rinsed, and then dried by heat at 130°C for 5 hours. The dried article thus obtainedwas milled with a jet mill to obtain titanium dioxide fine particle having a high-density silica coating layer on the surface thereof (an amount of the high-density silica coating layer was 58 weight % with respect to 100 weight % of the titanium dioxide).

Example 7 (not part of the invention)

Dimethylpolysiloxane (silicone oil KF-96 produced by Shin-Etsu Chemical Co., Ltd.) was sprayed onto the powder of the titanium dioxide fine particle prepared in Example 5 in an amount 10 weight % with respect to 100 weight % of the above titanium dioxide fine particle while stirring the powder of the titanium dioxide fine particle with a super mixer to obtain titanium dioxide fine particle treated with dimethylpolysiloxane.

Example 8 (not part of the invention)

Branched silicone (silicone oil KF-9909 produced by Shin-Etsu Chemical Co., Ltd.) was sprayed onto the powder of the titanium dioxide fine particle prepared in Example 5 in an amount 10 weight % with respect to 100 weight % of the above titanium dioxide fine particle while stirring the powder of the titanium dioxide fine particle with a super mixer to obtain titanium dioxide fine particle treated with branched silicone.

Example 9 (not part of the invention)

Dimethylpolysiloxane (silicone oil KF-96 produced by Shin-Etsu Chemical Co., Ltd.) was sprayed onto the powder of the titanium dioxide fine particle prepared in Example 5 in an amount 6 weight % with respect to 100 weight % of the above titanium dioxide fine particle while stirring the powder of the titanium dioxide fine particle with a super mixer to treat the powder with dimethylpolysiloxane, and then branched silicone (silicone oil KF-9909 produced by Shin-Etsu Chemical Co., Ltd.) was sprayed in an amount 4 weight % with respect to 100 weight % of the above titanium dioxide fine particle to obtain titanium dioxide fine particle treated with dimethylpolysiloxane and branched silicone.

Comparative Example 4

An aqueous suspension of titanium dioxide fine particle was prepared by the same method as in Example 5 and to this, an aqueous solution of sodium aluminate in an amount of 30 weight % in terms of $Al_2O_3$ with respect to 100 weight % of the above titanium dioxide fine particle was added at a temperature within a range of 25 to 30°C, and sulfuric acid was added over 180 minutes to neutralize the mixture to yield a pH of 8.5. After this, titanium dioxide fine particle having a coating layer consisting of hydrous aluminum oxide was obtained by following the same procedure as in Example 5.

Comparative Example 5

Dimethylpolysiloxane (silicone oil KF-96 produced by Shin-Etsu Chemical Co., Ltd.) was sprayed onto the titanium dioxide fine particle prepared in Comparative Example 4 in an amount 10 weight % with respect to 100 weight % of the titanium dioxide fine particle while stirring the titanium dioxide fine particle with a super mixer to obtain titanium dioxide fine particle treated with dimethylpolysiloxane.

Comparative Example 6

The titanium dioxide fine particle prepared in Example 1 was dried by heat at 110°C for 5 hours to obtain the powder of titanium dioxide fine particle. Methylhydrogenpolysiloxane (silicone oil KF-99 produced by Shin-Etsu Chemical Co., Ltd.) was sprayed onto the obtained powder in an amount 10 weight % with respect to 100 weight % of the titanium dioxide fine particle while stirring the obtained powder with a super mixer to obtain titanium dioxide fine particle treated with methylhydrogen polysiloxane.

(Method of testing light stability)

0.7 g of the titanium dioxide fine particle obtained in Examples 5 and 6 and Comparative Example 4 described above and 6. 3 g of white Vaseline (the Japanese Pharmacopoeia) were kneaded with a Hoover type muller and then the mixture was placed in a polyethylene container which was 30 mm in inner diameter and 10 mm in depth to form a specimen. Ultraviolet rays (365 nm) were irradiated to this specimen using a portable ultraviolet lamp (UVGL-25 type manufactured by Ultraviolet Products) and a change in color difference $\Delta E$ corresponding to an irradiation time was measured. And, the cake of the titanium dioxide fine particle obtained in Example 1 was dried at 110°C for 5 hours, and on the resulting powder of the titanium dioxide fine particle, the same procedure was performed and a change in color difference $\Delta E$ corresponding to an irradiation time was measured.

As for the color difference, values of L, a and b in a hunter system were measured before and after irradiation

with a colorimeter (SM-5 type manufactured by Suga Test Instruments Co., Ltd.) and color difference $\Delta E$ between pre-irradiation and post-irradiation was determined by a calculation based on measurements. When a value of the color difference is small, this indicates that light stability is excellent. The results are shown in Table 2. The relationship between an ultraviolet irradiation time and a color difference $\Delta E$ is shown as a graph in Fig. 1. And, the value of the color difference $\Delta E$ was determined from an equation $\Delta E = (\Delta L^2 + \Delta a^2 + \Delta b^2)^{1/2}$.

Table 2

| | $\Delta E$ | | | |
|---|---|---|---|---|
| Time (HR) | Example 5 | Example 6 | Comparative Example 4 | Example 1 |
| 0 | 0 | 0 | 0 | 0 |
| 2 | 2.4 | 1.4 | 3.9 | 10.1 |
| 4 | 4.3 | 2.1 | 7.7 | 17.8 |
| 8 | 5.2 | 3.8 | 9.8 | 20.1 |
| 12 | 5.4 | 3.8 | 10.6 | 20.9 |
| 24 | 6.0 | 4.0 | 11.1 | 24.0 |
| 36 | 6.5 | 4.1 | 11.7 | 25.4 |
| 48 | 7.0 | 4.1 | 12.3 | 27.1 |

As is apparent from Table 2, Vaseline mixed with the titanium dioxide fine particle, having a high-density silica coating layer, of Examples 5 and 6 had a small degree of yellowing. Accordingly, it is apparent that the photo catalytic power was significantly inhibited in the titanium dioxide fine particle having a high-density silica coating layer in accordance with the present disclosure. And, a degree of yellowing was improved even in the titanium dioxide fine particle, having a coating layer consisting of hydrous aluminum oxide, of Comparative Example 4 compared with that of Example 1.

(Preparation of emulsion)

A cosmetic composition containing the titanium dioxide fine particle of Example 7 and Comparative Examples 5 and 6 described above was prepared by following the procedure described below. An emulsion was prepared using ULTRA-TURRAX T-25 manufactured by IKA Japan K.K. A mixed solution having formulation shown in A in Table 3 was prepared and dispersed at 1200 rpm for 3 minutes to form an oil phase A. Next, a mixed solution having formulation shown in B in Table 3 was prepared and dispersed at 1200 rpm for 3 minutes to form a water phase B. The water phase B was added to the oil phase A and the resulting mixture was dispersed at 1200 rpm for 3 minutes to prepare an emulsion.

Table 3

| | Material | Amount to be mixed (weight %) |
|---|---|---|
| A | Powder of titanium oxide fine particle composition | 10.0 |
| | KF-995 | 5.0 |
| | isohexadecane | 4.0 |
| | Cremophor WO7 | 1.5 |
| | Cremophor A25 | 0.2 |
| | cetyl alcohol | 0.5 |
| B | Simulgel NS | 1.5 |
| | xanthan gum | 0.5 |
| | pure water | 76.8 |

(Evaluation of photo activity)

The emulsions derived from Example 7 and Comparative Examples 5 and 6 obtained by the above-mentioned

method were exposed to direct sunlight for 2 hours outdoors in a fine day in a state of being contained in a sealed transparent glass container. A change in color (photochromism) by light irradiation was visually observed. The results of observations are shown in Table 4.

Table 4

| | Color before irradiation | Color after irradiation |
|---|---|---|
| Example 7 | white | white |
| Comparative Example 5 | white | blue |
| Comparative Example 6 | white | blue |

As is apparent from Table 4, an emulsion mixed with the titanium dioxide fine particle, which has a high-density silica coating layer and is treated with dimethylpolysiloxane, did not cause photochromism (Example 7). On the other hand, photochromism occurred in the titanium dioxide fine particle which has a coating layer consisting of hydrous aluminum oxide and is treated with dimethylpolysiloxane and the titanium dioxide fine particle which is treated with methylhydrogenpolysiloxane (Comparative Examples 5 and 6). Accordingly, the photo activity was significantly inhibited in the titanium dioxide fine particle which has a high-density silica coating layer and is treated with dimethylpolysiloxane in accordance with the present disclosure.

(Evaluation of feeling)

A cosmetic composition containing the titanium dioxide fine particle of Examples 7, 8 and 9 described above was prepared by following the procedure described below. An emulsion was prepared using ULTRA-TURRAX T-25 manufactured by IKA Japan K. K. A mixed solution having formulation shown in A in Table 5 was prepared and dispersed at 1200 rpm for 3 minutes to form an oil phase A. Next, a mixed solution having formulation shown in B in Table 5 was prepared and dispersed at 1200 rpm for 3 minutes to form a water phase B. The water phase B was added to the oil phase A and the resulting mixture was dispersed at 1200 rpm for 3 minutes to prepare an emulsion. A feeling of the emulsion was rated according to the following criteria by ten raters and an average score was determined. The results of evaluations are shown in Table 6.

(criteria of rating)

good: 4, rather good: 3, ordinary: 2, rather bad: 1, bad: 0

Table 5

| | Material | Amount to be mixed (weight %) |
|---|---|---|
| A | Powder of titanium oxide fine particle composition | 10.0 |
| | KF-995 | 27.0 |
| | KF-6017 | 3.0 |
| | KF-96-6cs | 20.0 |
| | CETIOL ININ | 5.0 |
| B | Pure water | 30.0 |
| | 1,3-butylene glycol | 5.0 |

Table 6

| | Good (4) | Rather good (3) | Ordinary (2) | Rather bad (1) | Bad (0) | Average score |
|---|---|---|---|---|---|---|
| Example 7 | 0 person | 0 person | 4 persons | 4 persons | 2 persons | 1.2 |
| Example 8 | 3 persons | 2 persons | 5 persons | 0 person | 0 person | 2.8 |
| Example 9 | 0 person | 2 persons | 6 persons | 2 persons | 0 person | 2.0 |

As is apparent from Table 6, an emulsion mixed with the titanium dioxide fine particle, which has branched silicone in the coating layer of an organic silicon compound, has a better feeling than an emulsion mixed with the titanium dioxide fine particle having straight chain silicone in the coating layer of an organic silicon compound, and therefore in the titanium dioxide fine particle having a coating layer of branched silicone in accordance with the present disclosure, frictional feeling of the emulsion resulting from a high-density silica coating layer is significantly improved.
Commercially available articles used in Tables 3 and 5 are as follows.
KF-995: Cyclic dimethyl silicone oil (produced by Shin-Etsu Chemical Co., Ltd.)
KF-6017: Polyether modified silicone (produced by Shin-Etsu Chemical Co., Ltd.)
KF-96-6cs: Dimethyl silicone oil (produced by Shin-Etsu Chemical Co., Ltd.)
Cremophor WO7: PEG-7 hydrogenated castor oil (produced by BASF)
Cremophor A25: Ceteareth-25 (produced by BASF)
Simulgel NS: Mixed solution of hydroxyethyl acrylate-sodium acryloyldimethyltaurate copolymer, squalane, polysorbate 60 and water (produced by SEIWA KASEI Co., Ltd.)
CETIOL ININ: Isononyl isononanoate (produced by Cognis Japan Ltd.)

INDUSTRIAL APPLICABILITY

In accordance with the production method of the present invention, titanium dioxide fine particle of 5 to 15 nm in a particle diameter having a high ultraviolet protection property and high transparency for visible light can be produced at low price. In the case where the rutile titanium dioxide fine particle of the present disclosure, which has the high-density silica coating layer, is mixed in cosmetic compositions, deterioration of other ingredients in cosmetic compositions is suppressed due to its low optical activity. Thus, the rutile titanium dioxide fine particle of the present disclosure, which has a high density silica coating layer, is suitably applicable to sun block compositions such as sunscreen cosmetic compositions, UV shielding cosmetic compositions such as makeup bases and foundations.
The powder, which is formed by treating the titanium dioxide fine particle having a high-density silica coating layer with the branched silicone, performs efficacy of improving frictional feeling resulting from a high-density silica coating layer and can be employed as the above cosmetic composition.

## Claims

1. A method of producing a rutile titanium dioxide fine particle having an average primary particle diameter of 5 to 15 nm, comprising the steps of

   (1) treating titanium dioxide hydrate with at least one of a basic compound selected from the group consisting of hydroxides of alkali metal and hydroxides of alkaline-earth metal, and (2) treating the compound obtained by the step (1) with a carboxylic acid group-containing compound and an inorganic acid,
   step 2) including a method in which a carboxylic group-containing compound is added to a suspension of the compound obtained by the step (1) thereby creating a mixture, heating of this mixture is initiated, and an inorganic acid is added to this mixture when a mixture temperature reaches 60°C or higher, wherein the carboxylic acid group-containing compound is used in an amount 0.25 to 1.5 mole% with respect to 100 mole% of $TiO_2$.

## Patentansprüche

1. Verfahren zur Herstellung eines Rutiltitandioxid-Feinpartikels mit einem durchschnittlichen Primärpartikeldurchmesser von 5 bis 15 nm, umfassend die folgenden Schritte:

   (1) Behandeln des Titandioxidhydrats mit wenigstens einer basischen Verbindung, die aus der Gruppe, die aus Alkalimetallhydroxiden und Erdalkalimetallhydroxiden besteht, gewählt ist, und (2) Behandeln der durch Schritt (1) erhaltenen Verbindung mit einer eine Carbonsäuregruppe enthaltenden Verbindung und einer anorganischen Säure,
   wobei Schritt (2) ein Verfahren umfasst, bei dem eine eine Carbonsäuregruppe enthaltende Verbindung zu einer Suspension aus der durch Schritt (1) erhaltenen Verbindung hinzugefügt wird, wodurch eine Mischung entsteht, ein Erwärmen dieser Mischung eingeleitet wird und eine anorganische Säure zu dieser Mischung hinzugefügt wird, wenn eine Mischungstemperatur 60°C oder mehr erreicht, wobei die eine Carbonsäuregruppe enthaltende Verbindung in einer Menge von 0,25 bis 1,5 Mol% in Bezug auf 100 Mol% von $TiO_2$ verwendet wird.

**Revendications**

1. Procédé de production d'une fine particule de dioxyde de titane rutile ayant un diamètre moyen de particule primaire de 5 à 15 nm, comprenant les étapes de

   (1) traitement de dihydrate de dioxyde de titane avec au moins un d'un composé basique choisi dans le groupe constitué d'hydroxydes de métal alcalin et d'hydroxydes de métal alcalino-terreux, et (2) de traitement du composé obtenu par l'étape (1) avec un composé contenant un groupe acide carboxylique et un acide inorganique, l'étape (2) comprenant un procédé dans lequel un composé contenant un groupe carboxylique est ajouté à une suspension du composé obtenu par l'étape (1) créant par-là un mélange, le chauffage de ce mélange est initié, et un acide inorganique est ajouté à ce mélange lorsqu'une température de mélange atteint 60°C ou supérieure, dans lequel le composé contenant un groupe acide carboxylique est utilisé dans une quantité de 0,25 à 1,5 % en mole par rapport à 100 % en mole de $TiO_2$.

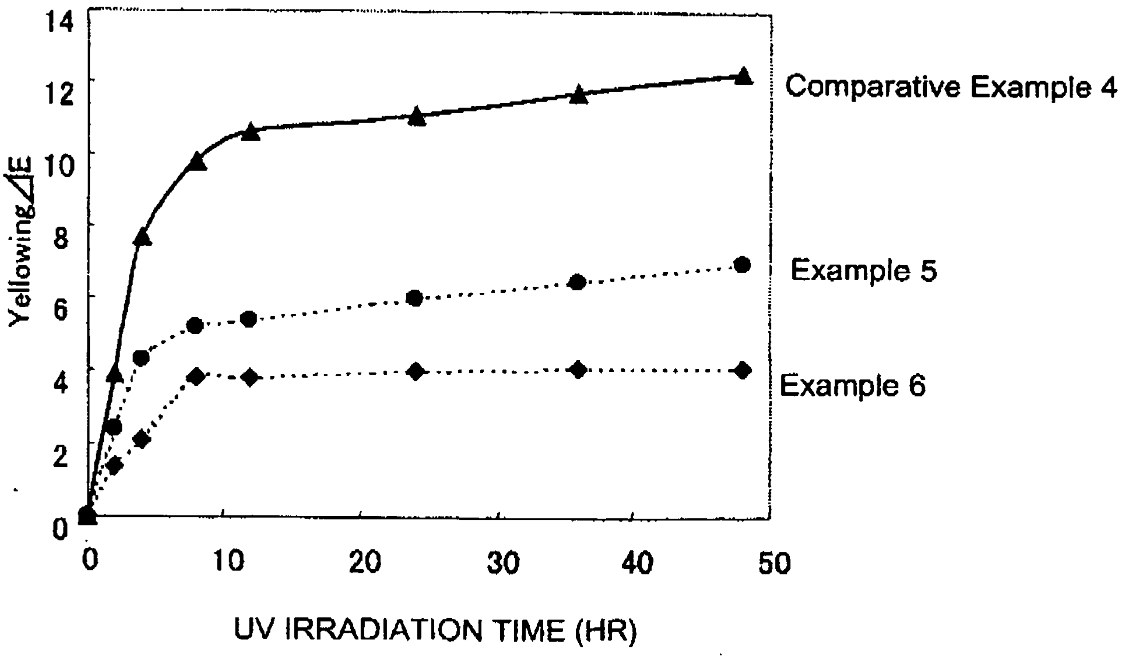
14
12
10
8
6
4
2
0
Yellowing⊿E
0
10
20
30
40
50
UV IRRADIATION TIME (HR)
Comparative Example 4
Example 5
Example 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6076215 B
- JP 2004115342 A
- JP 2002154917 A